(19) Europäisches Patentamt
European Patent Office
Office européen des brevets

(11) Publication number: 0 109 628
A1

(12) EUROPEAN PATENT APPLICATION

(21) Application number: 83111303.0

(22) Date of filing: 11.11.83

(51) Int. Cl.³: C 07 D 213/64
C 07 D 401/04, A 61 K 31/44
//C07D213/57, C07D213/55,
(C07D401/04, 213/00, 211/00)

(30) Priority: 18.11.82 US 442623
22.11.82 US 443249

(43) Date of publication of application:
30.05.84 Bulletin 84/22

(84) Designated Contracting States:
AT BE CH DE FR GB IT LI NL SE

(71) Applicant: STERLING DRUG INC.
90 Park Avenue
New York New York(US)

(72) Inventor: Lesher, George Yohe
R.D. 1, Box 268 Miller Road
East Greenbush New York(US)

(72) Inventor: Singh, Baldev
3 Blue Mountain Trail
East Greenbush New York(US)

(72) Inventor: Carabateas, Philip Michael
Box 432, R.D. 1
Nassau New York(US)

(74) Representative: Baillie, Iain Cameron et al,
c/o Ladas & Parry Isartorplatz 5
D-8000 München 2(DE)

# (54) 2(1H)-pyridinones and preparation, useful as cardiotonics.

(57) 3,4-Dihydro-3-$R_1$-4-$R_2$-5-Q-6-R-2 (1H)-pyridinones (I), where $R_1$ and $R_2$ are each hydrogen or methyl, R is lower-alkyl, and Q is 4 (or 3)-hydroxyphenyl, 4 (or 3)-methoxyphenyl, 4 (or 3)-pyridinyl or 4 (or 3)-pyridinyl having one or two lower-alkyl substituents, or acid-addition salts thereof, as well as the corresponding 3-$R_1$-4-$R_2$-5-Q-6-R-2 (1H)-pyridinones where at least one of $R_1$ and $R_2$ is methyl. The compounds have cardiotonic use when Q is 4 (or 3)-hydroxyphenyl, 4 (or 3)-pyridinyl or 4 (or 3)-pyridinyl having one or two lower-alkyl substituents. Said compounds are prepared from butanenitriles, butanoates, acrylamides or mixtures of alkanones and acetoacetamide, with optional conversion of the 3,4-dihydro compounds obtained to the 3,4-unsaturated compounds by heating with sulfur in an inert solvent and conversion of the methoxyphenyl compounds to the hydroxyphenyl compounds.



Croydon Printing Company Ltd.

This invention relates to 5-(pyridinyl or phenyl)-2(1H)-pyridinone derivatives useful as cardiotonics, and their preparation.

U.S. Patent 3,718,743 shows "5-phenyl-2-piperidinones and 5-phenyl-2-thiopiperidinones in compositions and methods for treating pain, fever and inflammation". The generic teaching of these piperidinones shows that "phenyl" can have one or two substituents at positions 2, 3, 4, 5 and/or 6, including alkyl, halogen, haloalkyl, aryl, nitro, amino, acylamino, acyl, carboxy, carbalkoxy, carbamyl, dialkylsulfamyl, alkylamino, dialkylamino, alkylmercapto, alkylsulfinyl and alkylsulfonyl. Various means of preparing the 5-phenyl-2-piperidinone final products are shown. In one procedure, a 2-chloro-5-phenylpyridine was heated with aqueous sodium hydroxide in dimethylformamide to produce the corresponding 5-phenyl-2(1H)-pyridinones which were then hydrogenated to produce the desired 5-phenyl-2-piperidinones. Among the intermediate 5-phenyl-2(1H)-pyridinones specifically shown is 5-(4-hydroxyphenyl)-2(1H)-pyridinone as well as its preparation by heating the corresponding 5-(4-methoxyphenyl)-2(1H)-pyridinone with pyridine hydrochloride under nitrogen.

U.S. Patents 4,004,012 and 4,072,746 show *inter alia*, as cardiotonic agents 5-(pyridinyl)-2(1H)-pyridinones

and their preparation by decarboxylating the corresponding 3-carboxy compounds. The disclosure of U.S. Patent 4,072,746 also is shown in U.S. Patents 4,107,315, 4,137,233, 4,199,586 and 4,225,715.

U.S. Patent 4,312,875, shows, inter alia, as cardiotonic agents 5-(pyridinyl)-6-(lower-alkyl)-2(1H)-pyridinones and their preparation by decarboxylating the corresponding 3-carboxy compounds.

5-(Hydroxyphenyl)-6-(lower-alkyl)-2(1H)-pyridinones and their cardiotonic use are disclosed in European Patent Publication 74091 published March 16, 1983.

The invention resides in a 3,4-dihydro-3-$R_1$-4-$R_2$-5-Q-6-R-2(1H)-pyridinone or 3-$R_1$-4-$R_2$-5-Q-6-R-2(1H)-pyridinone having Formula I

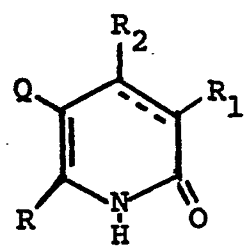

I

where there is either a single bond between the 3- and 4-positions of the pyridinone ring with hydrogen atoms at said positions (3,4-dihydro) or a double bond between said positions, Q is 4(or 3)-hydroxyphenyl, 4(or 3)-methoxyphenyl, 4(or 3)-pyridinyl or 4(or 3)-pyridinyl having one or two lower-alkyl substituents, $R_1$ and $R_2$ are each hydrogen or methyl but in the event that there is a double bond between the 3- and 4-positions, at least one of $R_1$ and $R_2$ is methyl and R is lower-alkyl, or an acid-addition salt thereof when Q is one of the pyridinyl radicals defined above. The compounds of

Formula I where Q is 4(or 3)-hydroxyphenyl, 4(or 3)-pyridinyl or 4(or 3)-pyridinyl having one or two lower-alkyl substituents are useful as cardiotonics, as determined by standard pharmacological evaluation procedures. The compounds of Formula I where Q is 4(or 3)-methoxyphenyl are useful as intermediates for preparing the cardiotonics of Formula I where Q is 4(or 3)-hydroxyphenyl. Preferred embodiments are 3,4-dihydro compounds of Formula I where Q is 4(or 3)-pyridinyl, R is methyl or ethyl, and at least one of $R_1$ or $R_2$ is methyl, and the compounds of Formula I where there is a double bond between the 3- and 4-positions, $R_1$ is methyl and $R_2$ is hydrogen.

A cardiotonic composition for increasing cardiac contractility comprises a pharmaceutically acceptable carrier and, as the active component thereof, a cardiotonically effective amount of the compound of Formula I where R, $R_1$ and $R_2$ are defined as in Formula I and Q is 4(or 3)-hydroxyphenyl, 4(or 3)-pyridinyl or 4(or 3)-pyridinyl having one or two lower alkyl substituents, or pharmaceutically acceptable acid-addition salt thereof when Q is a pyridinyl substituent.

One can increase cardiac contractility in a patient requiring such treatment by administering orally or parenterally in a solid or liquid dosage form to such patient a composition comprising a pharmaceutically acceptable carrier and, as active component thereof, a cardiotonically effective amount of the compound of Formula I where R, $R_1$ and $R_2$ are defined as in Formula I and Q is

4(or 3)-hydroxyphenyl, 4(or 3)-pyridinyl or 4(or 3)-pyridinyl having one or two lower-alkyl substituents, or pharmaceutically acceptable salt thereof when Q is a pyridinyl substituent.

One can prepare the 3,4-dihydro compound of Formula I by reacting a 1-Q-2-alkanone of the formula $Q-CH_2-C(=O)R$ (II) with an $\alpha$-$R_1$-$\beta$-$R_2$-acrylonitrile of the formula $R_2-CH=C(R_1)CN$ (III) in the presence of a strong base to produce 2-$R_1$-3-$R_2$-4-Q-4-(RCO)butanenitrile having Formula IV

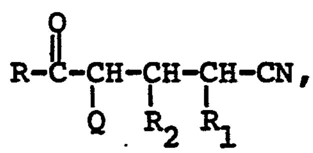 IV

reacting IV with a strong acid to produce 3,4-dihydro-3-$R_1$-4-$R_2$-5-Q-6-R-2(1H)-pyridinone having Formula I above, where $R_1$ and $R_2$ are each hydrogen or methyl, R is lower-alkyl, and Q is 4(or 3)-methoxyphenyl, 4(or 3)-pyridinyl or 4(or 3)-pyridinyl having one or two lower-alkyl substituents and, if desired, converting the compound having Formula I where Q is 4(or 3)-methoxyphenyl to the corresponding compound having Formula I where Q is 4(or 3)-hydroxyphenyl. Preferably, the strong base in the first step is an alkali hydride or methoxide, and the strong acid in the second step is a strong mineral acid.

Another process for preparing a 3,4-dihydro compound of Formula I comprises reacting a 1-Q-2-alkanone (II <u>supra</u>) with a lower-alkyl $\alpha$-$R_1$-$\beta$-$R_2$-acrylate of the formula $R_2-CH=C(R_1)COOR_3$ (V) to produce lower alkyl 2-$R_1$-3-$R_2$-4-Q-4-(RCO)butanoate having the Formula VI

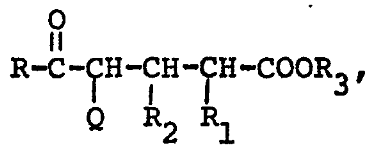

VI

reacting VI with ammonia or source thereof to produce 3,4-dihydro-3-$R_1$-4-$R_2$-5-Q-6-R-2(1H)-pyridinone having Formula I above, where $R_1$ and $R_2$ are each hydrogen or methyl, R and $R_3$ are each lower alkyl, and Q is 4(or 3)-methoxyphenyl, 4(or 3)-pyridinyl or 4(or 3)-pyridinyl having one or two lower-alkyl substituents, and, if desired, converting the compound having Formula I where Q is 4(or 3)-methoxyphenyl to the compound having Formula I where Q is 4(or 3)-hydroxyphenyl. Preferably, $R_3$ in the first step is methyl or ethyl and ammonium acetate is used as the source of ammonia in the second step.

Another process for preparing a 3,4-dihydro compound of Formula I comprises reacting a 1-Q-2-alkanone (II) with an α-$R_1$-β-$R_2$-acrylamide of the formula $R_2$-CH=C($R_1$)$CONH_2$ (VII) in an inert solvent in the presence of a strong base, preferably an alkali metal tertiary-butoxide, to produce 3,4-dihydro-3-$R_1$-4-$R_2$-5-Q-6-R-2(1H)-pyridinone having Formula I, where $R_1$ and $R_2$ are each hydrogen or methyl, R is lower-alkyl, and Q is 4(or 3)-methoxyphenyl, 4(or 3)-pyridinyl or 4(or 3)-pyridinyl having one or two lower-alkyl substituents, and, if desired, converting the compound having Formula I where Q is 4(or 3)-methoxyphenyl to the compound having Formula I where Q is 4(or 3)-hydroxyphenyl. Preferably dioxane and potassium or sodium tertiary-butoxide are used in the first step.

One can heat 3,4-dihydro-3-$R_1$-4-$R_2$-5-Q-6-R-2(1H)-pyridinone having the Formula IA

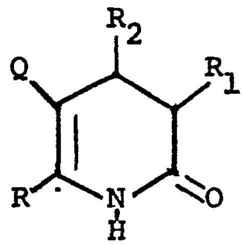

IA

in a suitable inert solvent in the presence of sulfur to produce 3-$R_1$-4-$R_2$-5-Q-6-R-2(1H)-pyridinone having the Formula IB

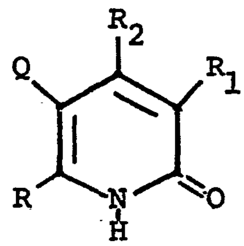

IB

where $R_1$ and $R_2$ are each hydrogen or methyl, R is lower-alkyl, and Q is 4(or 3)-methoxyphenyl, 4(or 3)-hydroxyphenyl, 4(or 3)-pyridinyl or 4(or 3)-pyridinyl having one or two lower-alkyl substituents, and, if desired, converting the compound having Formula IB where Q is 4(or 3)-methoxyphenyl to the corresponding compound having Formula IB where Q is 4(or 3)-hydroxyphenyl. Preferably the inert solvent is a eutectic mixture of diphenyl and diphenyl ether (DOWTHERM® A). Said process is also applicable to the preparation of compounds of the Formula IA where $R_1$ and $R_2$ are both hydrogens, thus including prior art compounds acknowledged above. However, said process is preferably carried out to prepare novel compounds of the invention where at least one of $R_1$ or $R_2$ is methyl.

Another process for preparing the 3,4-unsaturated compounds of Formula IB comprises heating a mixture of a 1-Q-2-alkanone of the Formula Q-$CH_2$-C(=O)R (II) and

acetoacetamide with polyphosphoric acid to produce 4-methyl-5-Q-6-R-2(1H)-pyridinone (IB, $R_1$ is hydrogen and $R_2$ is methyl), where R is lower-alkyl, Q is 4(or 3)-methoxyphenyl, 4(or 3)-pyridinyl or 4(or 3)-pyridinyl having one or two lower-alkyl substituents, and, if desired, converting said 4-methyl-5-Q-6-R-2(1H)-pyridinone where Q is 4(or 3)-methoxyphenyl to the corresponding compound where Q is 4(or 3)-hydroxyphenyl. Preferably the reaction is run also using methanesulfonic acid in addition to the polyphosphoric acid.

The term "lower-alkyl", as used herein to define R, $R_3$ or the substituents of (4 or 3)-pyridinyl, means alkyl radicals having from one to four carbon atoms which

can be arranged as straight or branched chains, illustrated by methyl, ethyl, n-propyl, isopropyl, n-butyl, 2-butyl or isobutyl.

Illustrative of Q in formulas I, II, IV or VI where Q is 4- or 3-pyridinyl having one or two lower-alkyl substituents are the following: 2-methyl-4-pyridinyl, 2,6-dimethyl-4-pyridinyl, 3-methyl-4-pyridinyl, 2-methyl-3-pyridinyl, 6-methyl-3-pyridinyl (alternatively named 2-methyl-5-pyridinyl), 2,3-dimethyl-4-pyridinyl, 2,6-dimethyl-4-pyridinyl, 2-ethyl-4-pyridinyl, 2-isopropyl-4-pyridinyl, 2-n-butyl-4-pyridinyl, 2,6-diethyl-4-pyridinyl, 2,6-diethyl-3-pyridinyl, 2,6-diisopropyl-4-pyridinyl, and the like.

The basic compounds of the invention having formula I where Q is a pyridinyl substituent are useful both in the free base and in the form of acid-addition salts, and both forms are within the purview of the invention. The acid-addition salts are simply a more convenient form for use; and in practice, use of the salt form inherently amounts to use of the base form. The acids which can be used to prepare the acid-addition salts include preferably those which produce, when combined with the free base, pharmaceutically acceptable salts, that is, salts whose anions are relatively innocuous to the animal organism in pharmaceutical doses of the salts, so that the beneficial cardiotonic properties inherent in the

free base form of the cardiotonically active compounds of the invention are not vitiated by side effects ascribable to the anions. In practicing the invention, it is convenient to use the free base form; however, appropriate pharmaceutically acceptable salts within the scope of the invention are those derived from mineral acids such as hydrochloric acid, sulfuric acid, phosphoric acid and sulfamic acid; and organic acids such as acetic acid, citric acid, lactic acid, tartaric acid, methanesulfonic acid, ethanesulfonic acid, benzenesulfonic acid, p-toluenesulfonic acid, cyclohexylsulfamic acid, quinic acid, and the like, giving the hydrochloride, sulfate, phosphate, sulfamate, acetate, citrate, lactate, tartrate, methanesulfonate, ethanesulfonate, benzenesulfonate, p-toluenesulfonate, cyclohexylsulfamate and quinate, respectively.

The acid-addition salts of said basic compounds of formula I are prepared either by dissolving the free base in aqueous or aqueous-alcohol solution or other suitable solvents containing the appropriate acid and isolating the salt by evaporating the solution, or by reacting the free base and acid in an organic solvent, in which case the salt separates directly or can be obtained by concentration of the solution.

Although pharmaceutically acceptable salts of said basic compounds are preferred, all acid-addition salts are within the scope of our invention. All acid-addition salts are useful as sources of the free base form even if the particular salt per se is desired only as an intermediate product as for example when the salt is formed only for purposes of purification or identification, or when it is used as an intermediate in preparing a pharmaceutically acceptable salt by ion exchange procedures.

The molecular structures of the compounds of the invention were assigned on the basis of evidence provided by infrared, nuclear magnetic resonance and mass spectra, and by the correspondence of calculated and found values for the elemental analyses.

The manner of making and using the instant invention will now be generally described so as to enable a person skilled in the art of pharmaceutical chemistry to make and use the same.

The reaction of a 1-Q-2-alkanone of the formula $Q-CH_2-C(=O)R$ (II) with an $\alpha$-$R_1$-$\beta$-$R_2$-acrylonitrile of the formula $R_2-CH=C(R_1)CN$ (III) to produce 2-$R_1$-3-$R_2$-4-Q-4-(RCO)butanenitrile (IV) is run by mixing the reactants at ambient temperature or in an ice bath in the presence of a strong base, such as an alkali hydride or alkaline metal hydride, preferably sodium hydride in mineral oil, or an alkali metal lower-alkoxide, preferably sodium methoxide in a lower-alkanol, preferably methanol. Other suitable strong bases include potassium

tert.-butoxide, potassium methoxide, sodamide, benzyltrimethylammonium hydroxide, tetramethylammonium hydroxide, 1,5-diazobicyclo[4.3.0]non-5-ene (DBN), and the like. Other suitable solvents include p-dioxane, dimethylformamide, tetrahydrofuran, hexamethylphosphortriamide, N-methylpyrrolidone, ethanol, dimethyl sulfoxide, and the like.

The reaction of the 1-Q-2-alkanone (II) with a lower-alkyl $\alpha$-$R_1$-$\beta$-$R_2$-acrylate of the formula $R_2$-CH=C($R_1$)$COOR_3$ (V) to produce lower-alkyl 2-$R_1$-3-$R_2$-4-Q-4-(RCO)butanoate of formula VI is conveniently carried by mixing the reactants at about 0°C. to 100°C., preferably at about 20°C. to 40°C., in the presence or absence of a suitable solvent such as ethanol, methanol, dimethylformamide, dimethyl sulfoxide, nitromethane, and the like. Optionally, the reaction can be expedited by adding a small quantity of a substance such as a 40% solution of benzyltrimethylammonium hydroxide in methanol.

The conversion of 2-$R_1$-3-$R_2$-4-Q-4-(RCO)butanenitrile (IV) to 3,4-dihydro-3-$R_1$-4-$R_2$-5-Q-6-R-2(1H)-pyridinone (I) is carried out by reacting IV with a strong acid in the absence or presence of a suitable solvent. For example, the reaction can be run by adding a strong mineral acid such as concentrated sulphuric acid to IV cooled in an ice bath and then allowing the reaction mixture to stand at room temperature alternatively the reaction can be run by bubbling hydrogen chloride gas into a solution of IV in a suitable solvent such as absolute ethanol or other lower alkanol. Also, this conversion can be carried out using as the strong acid polyphosphoric acid or concentrated phosphoric acid, preferably heating on a steam bath.

The conversion of lower-alkyl 2-$R_1$-3-$R_2$-4-Q-4-(RCO)butanoate (VI) to 3,4-dihydro-3-$R_1$-4-$R_2$-5-Q-6-R-2(1H)-pyridinone (I) is conveniently carried out by heating VI with ammonia or a source of ammonia such as ammonium acetate in a suitable solvent such as ethanol. The reaction can be run at about 0°C. to 100°C., preferably about 50°C. to 90°C. Other sources of ammonia include ammonium chloride, ammonium carbonate, ammonium formate, and the like. Optionally, the reaction can be run under pressure using ammonia. Other suitable solvents include methanol, dimethylformamide, dimethyl sulfoxide, p-dioxane, nitromethane, and the like.

The reaction of 1-Q-2-alkanone (II) with an α-$R_1$-β-$R_2$-acrylamide (VII) is conveniently run in an inert solvent and in the presence of a strong base, preferably an alkali metal <u>tertiary</u>-butoxide, preferably by mixing II, VII and potassium <u>tertiary</u>-butoxide in p-dioxane at ambient temperature and then heating if necessary up to about 100°C., to produce 3,4-dihydro-3-$R_1$-4-$R_2$-5-Q-6-R-2(1H)-pyridinone (I). Other suitable inert solvents and strong bases include those given above in the reaction of II and III to produce IV.

The conversion of the 3,4-dihydro-3-$R_1$-4-$R_2$-5-Q-6-R-2(1H)-pyridinone (IA) to the corresponding 3-$R_1$-4-$R_2$-5-Q-6-R-2(1H)-pyridinone (IB) is carried out by heating IA in a suitable inert solvent, preferably a eutectic mixture of diphenyl and diphenyl ether (Dowtherm A), in the presence of sulfur. Preferably about one mole of sulfur per one mole of the 3,4-dihydro-2(1H)-pyridinone is used. The reaction is carried out at about 150°C. to 250°C., preferably about 170°C. to 220°C. Other solvents that can be used include mineral oil, 1-methyl-2-pyrrolidinone, diethyl phthalate, and the like. Dimethyl sulfoxide can be used as solvent only when Q in IA is 4(or 3)-methoxyphenyl or 4(or 3)-hydroxyphenyl; and, tetramethylene sulfoxide can be used here in place of dimethyl sulfoxide.

The reaction of a 1-Q-2-alkanone (II) and acetoacetamide with polyphosphoric acid to produce 4-methyl-5-Q-6-R-2(1H)-pyridinone (IB, $R_1$ is hydrogen and $R_2$ is methyl) is carried out by heating the reactants at about 70°C. to 150°C., preferably about 110°C. to 130°C. Optionally and preferably, the reaction is run also using a mixture of polyphosphoric acid and methanesulfonic acid to make the reaction mixture more fluid.

The conversion of 3-$R_1$-4-$R_2$-5-Q-6-R-2(1H)-pyridinone or 3,4-dihydro-3-$R_1$-4-$R_2$-5-Q-6-R-2(1H)-pyridinone of Formula I as defined above where Q is 4(or 3)-methoxyphenyl to the corresponding compound (I) where Q is 4(or 3)-hydroxyphenyl is conveniently carried out by conventional means of cleaving a methoxyphenyl compound to produce the corresponding hydroxyphenyl compound, such as by heating a collidine solution of the methoxyphenyl compound (I or II) in the presence of lithium iodide. This conversion can be run by refluxing I or II where Q is 4(or 3)-methoxyphenyl with concentrated (48%) hydrobromic acid, concentrated (57%) hydriodic acid, concentrated (85%) phosphoric acid or with aluminum trichloride or boron trifluoride in toluene.

The following examples will further illustrate the invention without, however, limiting it thereto.

A. 2-$R_1$-3-$R_2$-4-Q-4-(RCO)BUTANENITRILES

A-1. 4-Acetyl-4-(4-pyridinyl)butanenitrile-To a 13.5 g portion of 1-(4-pyridinyl)-2-propanone cooled in an ice bath was added 50 mg of sodium hydride as a 50% suspension in mineral oil and the resulting mixture was stirred for about 10 minutes. To the stirred solution was added 5.3 g of acrylonitrile dropwise over a period of 20 minutes. The ice bath was removed and the mixture was stirred at room temperature overnight. To the mixture was added 0.5 ml of acetic acid, the resulting mixture was partitioned between water and chloroform

(150 ml of each) and the resulting mixture was shaken well. The two phases were separated and the chloroform phase was evaporated using a rotary evaporator to yield, as an oil, 17.5 g of material containing primarily 4-acetyl-4-(4-pyridinyl)butanenitrile, which was identified by its nmr spectrum and which was used in the next step without further purification.

A-2. 4-Acetyl-3-methyl-4-(4-pyridinyl)butanenitrile - To a stirred mixture mixture containing 59.6 g of 1-(4-pyridinyl)propanone and 100 ml of crotononitrile was added a solution of sodium methoxide in methanol obtained by adding 1 pellet of sodium shot to 20 ml of methanol. The reaction mixture was heated on a steam bath with stirring. After about 5 minutes, there was an exothermic reaction which raised the reaction temperature to 100°C. The resulting mixture was allowed to cool to room temperature and stand overnight (about 16 hours). To the reaction was added 100 ml of 10% acetic acid and 300 ml of methylene dichloride; and, the mixture was shaken well. The layers were separated and the methylene dichloride layer was dried over anhydrous magnesium sulfate and filtered. The methylene dichloride was distilled off *in vacuo* and the residual oil was distilled under vacuum using a small column to produce 85.0 g of 4-acetyl-3-methyl-4-(4-pyridinyl)butanenitrile, b.p. 130-135°C. at 0.05 mm.

Following the procedure described in Example A-1 or A-2 using in place of 1-(4-pyridinyl)propanone and acrylonitrile or crotononitrile molar equivalent quantities of the appropriate 1-Q-2-alkanone and $\alpha$-$R_1$-$\beta$-$R_2$-acrylonitrile, it is contemplated that the corresponding 2-$R_1$-3-$R_2$-4-Q-4-(RCO)butanenitriles of Examples A-3 through A-12 can be obtained.

A-3. <u>4-Acetyl-2,3-dimethyl-4-(4-pyridinyl)butanenitrile</u>, using 1-(4-pyridinyl)propanone and 2-methyl-2-butenenitrile.

A-4. <u>4-Acetyl-4-(3-pyridinyl)butanenitrile</u>, using 1-(3-pyridinyl)propanone and acrylonitrile.

A-5. <u>4-Acetyl-2-methyl-4-(4-pyridinyl)butanenitrile</u>, using 1-(4-pyridinyl)propanone and $\alpha$-methylacrylonitrile.

A-6. <u>1-Methyl-4-n-propanoyl-4-(4-pyridinyl)butanenitrile</u>, using 1-(4-pyridinyl)butanone and $\alpha$-methylacrylonitrile.

A-7. <u>1-Methyl-4-(2-methylpropanoyl)-4-(4-pyridinyl)butanenitrile</u>, using 1-(4-pyridinyl)-3-methyl-2-butanone and $\alpha$-methylacrylonitrile.

A-8. <u>2-Methyl-4-(n-pentanoyl)-4-(4-pyridinyl)butanenitrile</u>, using 1-(4-pyridinyl)-2-hexanone and $\alpha$-methylacrylonitrile.

A-9. <u>4-Acetyl-2,3-dimethyl-4-(4-pyridinyl)butanenitrile</u>, using 1-(2-methyl-4-pyridinyl)-2-propanone and 2-methyl-2-butenenitrile.

A-10. 2-Methyl-4-(2,6-dimethyl-4-pyridinyl)-4-n-propanoyl)butanenitrile, using 1-(2,6-dimethyl-4-pyridinyl)-2-butanone and α-methylacrylonitrile.

A-11. 4-Acetyl-4-(4-methoxyphenyl)-2-methylbutanenitrile, using 1-(4-methoxyphenyl)-2-propanone and α-methylacrylonitrile.

A-12. 2-Methyl-4-(3-methoxyphenyl)-4-n-propanoylbutanenitrile, using 1-(3-methoxyphenyl)-2-butanone and α-methylacrylonitrile.

B. LOWER-ALKYL 2-$R_1$-3-$R_2$-4-Q-4-(RCO)BUTANOATES

B-1. Ethyl 4-Acetyl-3-methyl-4-(4-pyridinyl)butanoate - A mixture containing 13.5 g of 1-(4-pyridinyl)-2-propanone, 20 ml of ethyl crotonates and 0.5 ml of Triton B (a 40% solution of benzyltrimethylammonium hydroxide in methanol) was heated on a steam bath for 2 hours, allowed to cool to room temperature and to stand overnight. There was thus obtained as an oil a mixture containing as a major portion thereof ethyl 4-acetyl-3-methyl-4-(4-pyridinyl)butanoate, which was identified by its mass spectrum. This material was used in the next step without further purification.

Following the procedure described in Example B-1 but using in place of 1-(4-pyridinyl)-2-propanone and ethyl crotonate, molar equivalent quantities of the appropriate 1-Q-2-alkanone and lower-alkyl α-$R_1$-β-$R_2$acrylate, it is contemplated that the corresponding lower-alkyl 2-$R_1$-3-$R_2$-4-Q-4-(RCO)butanoates of Examples B-2 through B-12 can be obtained.

B-2. Ethyl 4-Acetyl-4-(4-pyridinyl)butanoate, using 1-(4-pyridinyl)-2-propanone and ethyl acrylate.

B-3. Methyl 4-Acetyl-2-methyl-4-(4-pyridinyl)butanoate, using 1-(4-pyridinyl)-2-propanone and methyl α-methylacrylate.

B-4. Ethyl 4-Acetyl-2,3-dimethyl-4-(4-pyridinyl)-butanoate, using 1-(4-pyridinyl)-2-propanone and ethyl α,β-dimethylacrylate.

B-5. Ethyl 4-Acetyl-4-(3-pyridinyl)butanoate, using 1-(3-pyridinyl)-2-propanone and ethyl acrylate.

B-6. Ethyl 1-Methyl-4-n-propanoyl-4-(4-pyridinyl)-butanoate, using 1-(4-pyridinyl)-2-butanone and ethyl α-methylacrylate.

B-7. Ethyl 2-Methyl-4-(2-methyl-n-propanoyl)-4-(4-pyridinyl)butanoate, using 1-(4-pyridinyl)-3-methyl-2-butanone and ethyl α-methylacrylate.

B-8. Ethyl 4-n-butanoyl-2-methyl-4-(4-pyridinyl)-butanoate, using 1-(4-pyridinyl)-2-hexanone and ethyl α-methylacrylate.

B-9. Ethyl 4-Acetyl-2,3-dimethyl-4-(2-methyl-4-pyridinyl)butanoate, using 1-(2-methyl-4-pyridinyl)-2-propanone and ethyl α,β-dimethylacrylate.

B-10. Ethyl 2-Methyl-4-n-propanoyl-4-(2,6-dimethyl-4-pyridinyl)butanoate, using 1-(2,6-dimethyl-4-pyridinyl)-2-butanone and ethyl α-methylacrylate.

B-11. <u>Ethyl 4-Acetyl-4-(4-methoxyphenyl)-2-methyl butanoate</u>, using 1-(4-methoxyphenyl)-2-propanone and ethyl α-methylacrylate.

B-12. <u>Ethyl 4-(3-Methoxyphenyl)-4-n-propanoyl)-2-methyl butanoate</u>, using 1-(3-methoxyphenyl)-2-butanone and ethyl α-methylacrylate.

C. <u>3,4-DIHYDRO-3-$R_1$-4-$R_2$-5-Q-6-R-2(1H)-PYRIDINONES</u>

C-1. <u>3,4-Dihydro-6-methyl-5-(4-pyridinyl)-2(1H)-pyridinone</u>, alternatively named 4,5-dihydro-2-methyl-[3,4'-bipyridin]-6(1H)-one - To a 17 g portion of 4-acetyl-4-(4-pyridinyl)butanenitrile cooled in an ice bath was added slowly 50 ml of concentrated sulphuric acid while the mixture was shaken slowly. The reaction mixture was allowed to stand in the ice bath for 2 hours and then at room temperature overnight; it was then poured onto ice (600 ml beaker half filled) and the resulting aqueous mixture was neutralized by adding aqueous ammonium hydroxide. The oily material that separated solidified while the mixture was allowed to stand at room temperature for 3 hours. The solid was collected, washed with water and dried to yield 13.2 g of material which was purified chromatographically using 500 g of silica gel and 15% of methanol in ether as the eluant. The least polar component was collected and recrystallized from isopropyl alcohol-ether to produce 6.3 g of 3,4-dihydro-6-methyl-5-(4-pyridinyl)-2(1H)-pyridinone, m.p. 175-177°C.

Acid-addition salts of 3,4-dihydro-6-methyl-5-(4-pyridinyl)-2(1H)-pyridinone are conveniently prepared by adding to a mixture of 1 g of 3,4-dihydro-6-methyl-5-(4-pyridinyl)-2(1H)-pyridinone in about 20 ml of aqueous methanol the appropriate acid, e.g., hydrochloric acid, methanesulfonic acid, concentrated sulfuric acid, concentrated phosphoric acid, to a pH of about 2 to 3, chilling the mixture after partial evaporation and collecting the precipitated salt, e.g., hydrochloride, methanesulfonate, sulfate, phosphate, respectively. Also, the acid-addition salt is conveniently prepared in aqueous solution by adding to water with stirring a molar equivalent quantity each of 3,4-dihydro-6-methyl-5-(4-pyridinyl)-2(1H)-pyridinone and the appropriate acid, e.g., lactic acid or hydrochloric acid, to prepare respectively the lactate or hydrochloride salt of 3,4-dihydro-6-methyl-5-(4-pyridinyl)-2(1H)-pyridinone in aqueous solution.

C-2. 3,4-Dihydro-4,6-dimethyl-5-(4-pyridinyl)-2(1H)-pyridinone, alternatively named 4,5-dihydro-2,4-dimethyl-[3,4'-bipyridin]-6(1H)-one - To a 2.0 g portion of 4-acetyl-3-methyl-4-(4-pyridinyl)butanenitrile dissolved in 20 ml of acetic acid was added dropwise 3 ml of sulfuric acid whereupon an exothermic reaction ensued. The reaction mixture was stirred for 4 hours, the acetic acid was evaporated off *in vacuo* and to the remaining material was added 10 ml of water followed by basification with 35% aqueous sodium hydroxide solution. The alkaline solution was extracted four times with ethyl acetate; the combined extracts were dried over anhydrous magnesium sulfate; the ethyl acetate was distilled off *in vacuo*; and, the remaining white solid was recrystallized from acetonitrile to produce 2.0 g of 3,4-dihydro-4,6-dimethyl-5-(4-pyridinyl)-2(1H)-pyridinone, m.p. 184-186°C.

The same compound was prepared by the following procedure: Into a solution containing 81 g of 4-acetyl-3-methyl-4-(4-pyridinyl)butanenitrile dissolved in 250 ml of absolute ethanol was bubbled hydrogen chloride gas at ambient temperature with no cooling. After about 1 hour, a white solid began to crystallize. The reaction vessel was then stoppered and allowed to stand overnight at room temperature. The reaction mixture was evaporated to dryness *in vacuo* and the residue was dissolved in a small amount of water. The aqueous solution was made alkaline with concentrated ammonia hydroxide and the mixture was extracted with ethyl acetate. The ethyl acetate solution was dried over anhydrous magnesium sulfate, the mixture filtered and the filtrate evaporated *in vacuo*. The residue was slurried with acetonitrile and the solid, 5.4 g, was collected. This solid was combined with the product obtained as described in the immediately preceding paragraph along with the product obtained

hereinbelow in Example C-3 and the combined material was recrystallized from about 250 ml of acetonitrile to yield 9.9 g of 3,4-dihydro-4,6-dimethyl-5-(4-pyridinyl)-2(1H)-pyridinone, m.p. 186-188°C.

Acid-addition salts of 3,4-dihydro-4,6-dimethyl-5-(4-pyridinyl)-2(1H)-pyridinone are conveniently prepared by adding to a mixture of 1 g of 3,4-dihydro-4,6-dimethyl-5-(4-pyridinyl)-2(1H)-pyridinone in about 20 ml of aqueous methanol the appropriate acid, e.g., hydrochloric acid, methanesulfonic acid, concentrated sulfuric acid, concentrated phosphoric acid, to a pH of about 2 to 3, chilling the mixture after partial evaporation and collecting the precipitated salt, e.g., hydrochloride, methanesulfonate, sulfate, phosphate, respectively. Also, the acid-addition salt is conveniently prepared in aqueous solution by adding to water with stirring a molar equivalent quantity each of 3,4-dihydro-4,6-dimethyl-5-(4-pyridinyl)-2(1H)-pyridinone and the appropriate acid, e.g., lactic acid or hydrochloric acid, to prepare respectively the lactate or hydrochloride salt of 3,4-dihydro-4,6-dimethyl-5-(4-pyridinyl)-2(1H)-pyridinone in aqueous solution.

C-3. <u>3,4-Dihydro-4,6-dimethyl-5-(4-pyridinyl)-2(1H)-pyridinone</u> - A mixture containing 24 g of ethyl 4-acetyl-3-methyl-4-(4-pyridinyl)butanoate, 7 g of ammonium acetate and 50 ml of ethanol was refluxed for 8 hours

with stirring. The reaction mixture was then concentrated to dryness *in vacuo* and the remaining white solid was collected, washed with ethanol and dried to produce 1.8 g of 3,4-dihydro-4,6-dimethyl-5-(4-pyridinyl)-2(1H)-pyridinone, m.p. 184-185°C. It's nmr and mass spectrum were consistent with the assigned structure.

C-4. 3,4-Dihydro-3,6-dimethyl-5-(4-pyridinyl)-2(1H)-pyridinone, alternatively named 4,5-dihydro-2,5-dimethyl[3,4'-bipyridin]-6(1H)-one - A mixture containing 35 g of 1-(4-pyridinyl)-2-propanone, 25 g of α-methylacrylamide, 30 g of potassium-*tert*.-butoxide and 250 ml of p-dioxane was stirred at ambient temperature for 40 minutes during which time an exothermic reaction occurred. The reaction mixture was then heated on a steam bath for 90 minutes and stripped to dryness on a rotary evaporator. To the residue was added 200 ml of water and the aqueous mixture was acidified with acetic acid. The product was extracted with chloroform (two 300 ml portions) and the combined extracts stripped to dryness *in vacuo*. The gummy solid was dissolved in 200 ml of isopropyl alcohol, the hot solution treated with decolorizing charcoal and filtered, and the filtrate diluted with 300 ml of n-hexane. The crystalline precipitate was collected and dried in an oven at 80°C. to yield 25.4 g of 3,4-dihydro-3,6-dimethyl-5-(4-pyridinyl)-2(1H)-pyridinone, m.p. 148-150°C. A second crop of 4.3 g, m.p. 147-150, was obtained from the mother liquor. The nmr spectrum of the product was consistent with the assigned structure.

Acid-addition salts of 3,4-dihydro-3,6-dimethyl-5-(4-pyridinyl)-2(1H)-pyridinone are conveniently prepared by adding to a mixture of 1 g of 3,4-dihydro-3,6-dimethyl-5-(4-pyridinyl)-2(1H)-pyridinone in about 20 ml of aqueous methanol the appropriate acid, e.g., hydrochloric acid, methanesulfonic acid, concentrated sulfuric acid, concentrated phosphoric acid, to a pH of about 2 to 3, chilling the mixture after partial evaporation and collecting the precipitated salt, e.g., hydrochloride, methanesulfonate, sulfate, phosphate, respectively. Also, the acid-addition salt is conveniently prepared in aqueous solution by adding to water with stirring a molar equivalent quantity each of 3,4-dihydro-3,6-dimethyl-5-(4-pyridinyl)-2(1H)-pyridinone and the appropriate acid, e.g., lactic acid or hydrochloric acid, to prepare respectively the lactate or hydrochloride salt of 3,4-dihydro-3,6-dimethyl-5-(4-pyridinyl)-2(1H)-pyridinone in aqueous solution.

C-5. 3,4-Dihydro-5-(4-methoxyphenyl)-6-methyl-2(1H)-pyridinone, 13.9 g, m.p. 160-162°C., was prepared following the procedure described in Example C-4 using 50 g of 1-(4-methoxyphenyl)-2-propanone, 28.4 g of acrylamide, 44.8 g of potassium tert.-butoxide, 400 ml of p-dioxane, stirring the reaction mixture at ambient temperature for 1 hour, heating the reaction mixture on a steam bath for 1 hour and then, after isolating the product as in the preceding example, crystallizing it once from isopropyl alcohol and once from 200 ml of ethanol.

C-6. 3,4-Dihydro-5-(4-hydroxyphenyl)-6-methyl-2(1H)-pyridinone - A mixture containing 9 g of 3,4-dihydro-5-(4-methoxyphenyl)-6-methyl-2(1H)-pyridinone, 100 ml of collidine and 38 g of lithium iodide was heated under reflux for 28 hours and then concentrated to dryness on a rotary evaporator. The residue was dissolved in 100 ml of water and again concentrated to dryness on a rotary evaporator; this process was repeated twice to remove the last traces of collidine. The solid residue was dissolved in water and the aqueous solution acidified with concentrated hydrochloric acid. The gummy solid that separated on chilling was collected, washed with water, dried at room temperature, recrystallized from ether-isopropyl alcohol using decolorizing charcoal and then chromatographed over 100 g of silica gel using 5% methanol in ether as eluant to produce 3.1 g of 3,4-dihydro-5-(4-hydroxyphenyl)-6-methyl-2(1H)-pyridinone, m.p. 197-200°C.

C-7. 3,4-Dihydro-5-(4-methoxyphenyl)-3,6-dimethyl-2(1H)-pyridinone, 43.2 g, m.p. 135-136, was prepared following the procedure described in Example C-5 using 50 g of 1-(4-methoxyphenyl)-2-propanone, 34 g of α-methyl acrylamide, 44.8 g of potassium tert.-butoxide, 300 ml of p-dioxane and recrystallization from isopropyl alcohol-n-hexane.

C-8. 3,4-Dihydro-5-(4-hydroxyphenyl)-3,6-dimethyl-2(1H)-pyridinone, 6.6 g, m.p. 168-170°C., was prepared following the procedure described in Example C-6 using 23.1 g of 3,4-dihydro-5-(4-methoxyphenyl)-3,6-dimethyl-2(1H)-pyridinone, 200 ml of collidine, 75 g of lithium iodide and recrystallization from 250 ml of boiling ethanol using decolorizing charcoal.

Following the procedure described in Example C-1 using in place of 4-acetyl-4-(4-pyridinyl)butanenitrile a corresponding molar equivalent quantity of 2-$R_1$-3-$R_2$-4-Q-4-(RCO)butanenitrile or following the procedure described in Example C-3 using in place of ethyl 4-acetyl-3-methyl-4-(4-pyridinyl)butanoate a molar equivalent quantity of the appropriate lower-alkyl 2-$R_1$-3-$R_2$-4-Q-4-(RCO)butanoate, it is contemplated that the corresponding 3,4-dihydro-3-$R_1$-4-$R_2$-5-Q-6-R-2(1H)-pyridinones of Examples C-9 through C-17 can be obtained.

C-9. 3,4-Dihydro-3,4,6-trimethyl-5-(4-pyridinyl)-2(1H)-pyridinone, using 4-acetyl-2,3-dimethyl-4-(4-pyridinyl)butanenitrile or ethyl 4-acetyl-2,3-dimethyl-4-(pyridinyl)butanoate.

C-10. 3,4-Dihydro-6-methyl-5-(3-pyridinyl)-2(1H)-pyridinone, using 4-acetyl-4-(3-pyridinyl)butanenitrile or ethyl 4-acetyl-4-(3-pyridinyl)butanoate.

C-11. 6-Ethyl-3,4-dihydro-3-methyl-5-(4-pyridinyl)-2(1H)-pyridinone, using 2-methyl-4-n-propanoyl-4-(4-pyridinyl)butanenitrile or ethyl 2-methyl-4-n-propanoyl-4-(4-pyridinyl)butanoate.

C-12. 3,4-Dihydro-6-isopropyl-3-methyl-5-(4-pyridinyl)-2(1H)-pyridinone, using 2-methyl-4-(3-methyl-n-propanoyl)-4-(4-pyridinyl)butanenitrile or ethyl 2-methyl-4-(3-methyl-n-propanoyl)-4-(4-pyridinyl)butanoate.

C-13. 6-n-Butyl-3,4-dihydro-3-methyl-5-(4-pyridinyl)-2(1H)-pyridinone, using 2-methyl-4-(n-pentanoyl)-4-(4-pyridinyl)butanenitrile or ethyl 2-methyl-4-(n-pentanoyl)-4-(4-pyridinyl)butanoate.

C-14. 3,4-Dihydro-3,4,6-trimethyl-5-(2-methyl-4-pyridinyl)-2(1H)-pyridinone, using 4-acetyl-2,3-dimethyl-4-(2-methyl-4-pyridinyl)butanenitrile or ethyl 4-acetyl-2,3-dimethyl-4-(2-methyl-4-pyridinyl)butanoate.

C-15. 6-Ethyl-3,4-dihydro-3-methyl-5-(2,6-dimethyl-4-pyridinyl)-2(1H)-pyridinone, using 2-methyl-4-(2,6-dimethyl-4-pyridinyl)-4-n-propanoylbutanenitrile or ethyl 2-methyl-4-(2,6-dimethyl-4-pyridinyl)-4-n-propanoylbutanoate.

C-16. 3,4-Dihydro-5-(4-methoxyphenyl)-3,6-dimethyl-2(1H)-pyridinone, using 4-acetyl-4-(4-methoxyphenyl)-2-methylbutanenitrile or ethyl 4-acetyl-4-(4-methoxyphenyl)-2-methylbutanoate.

C-17. 6-Ethyl-3,4-dihydro-5-(3-methoxyphenyl)-3-methyl-2(1H)-pyridinone, using 4-(3-methoxyphenyl)-2-methyl-4-n-propanoylbutanenitrile or ethyl 4-(3-methoxyphenyl)-2-methyl-4-n-propanoylbutanoate.

C-18. 6-Ethyl-3,4-dihydro-5-(3-hydroxyphenyl)-3-methyl-2(1H)-pyridinone can be prepared following the procedure described in Example C-6 using in place of

3,4-dihydro-5-(4-methoxyphenyl)-3,6-dimethyl-2(1H)-pyridinone a molar equivalent quantity of the corresponding 3,4-dihydro-5-(3-methoxyphenyl)-3,6-dimethyl-2(1H)-pyridinone.

Following the procedure described in Example C-4 but using in place of 1-(4-pyridinyl)-2-propanone and α-methylacrylamide corresponding molar equivalent quantities of the respective 1-Q-2-alkanone and α-$R_1$-β-$R_2$-acrylamide, it is contemplated that the corresponding 3,4-dihydro-3-$R_1$-4-$R_2$-5-Q-6-R-2(1H)-pyridinones of Examples C-19 through C-23 can be obtained.

C-19. 3,4-Dihydro-3,4,6-trimethyl-5-(4-pyridinyl)-2(1H)-pyridinone, using 1-(4-pyridinyl)-2-propanone and α-β-dimethylacrylamide.

C-20. 3,4-Dihydro-3,6-dimethyl-5-(3-pyridinyl)-2(1H)-pyridinone, using 1-(3-pyridinyl)-2-propanone and α-methylacrylamide.

C-21. 3,4-Dihydro-3,6-dimethyl-5-(2-methyl-4-(pyridinyl)-2(1H)-pyridinone, using 1-(2-methyl-4-pyridinyl)-2-propanone and α-methylacrylamide.

C-22. 3,4-Dihydro-3,6-dimethyl-5-(2,6-dimethyl-4-pyridinyl)-2(1H)-pyridinone, using 1-(2,6-dimethyl-4-pyridinyl)-2-propanone and α-methylacrylamide.

C-23. 6-Ethyl-3,4-dihydro-3-methyl-5-(4-pyridinyl)-2(1H)-pyridinone, using 1-(4-pyridinyl)-2-butanone and α-methylacrylamide.

D. <u>3-$R_1$-6-$R_2$-5-Q-6-R-2(1H)-PYRIDINONES</u>

D-1. <u>3,6-Dimethyl-5-(4-pyridinyl)-2(1H)-pyridinone</u>, alternatively named 2,5-dimethyl-[3,4'-bipyridin]-6(1H)-one - A mixture containing 15.1 g of 3,6-dihydro-3,6-dimethyl-5-(4-pyridinyl)-2(1H)-pyridinone, 50 ml of an

eutectic mixture of diphenyl and diphenyl ether (Dowtherm A) and 2.5 g of sulfur powder was heated in an oil bath at 195-200°C. for 2 and 1/2 hours and then allowed to cool to room temperature. The crystalline precipitate was collected, washed with ether and dried in a vacuum oven at 100°C. to yield 14.6 g of 3,6-dimethyl-5-(4-pyridinyl)-2(1H)-pyridinone, m.p. 256-258°C.

Acid-addition salts of 3,6-dimethyl-5-(4-pyridinyl)-2(1H)-pyridinone are conveniently prepared by adding to a mixture of 1 g of 3,6-dimethyl-5-(4-pyridinyl)-2(1H)-pyridinone in about 20 ml of aqueous methanol the appropriate acid, e.g., hydrochloric acid, methanesulfonic acid, concentrated sulfuric acid, concentrated phosphoric acid, to a pH of about 2 to 3, chilling the mixture after partial evaporation and collecting the precipitated salt, e.g., hydrochloride, methanesulfonate, sulfate, phosphate, respectively. Also, the acid-addition salt is conveniently prepared in aqueous solution by adding to water with stirring a molar equivalent quantity of 3,6-dimethyl-5-(4-pyridinyl)-2(1H)-pyridinone and the approriate acid, e.g., lactic acid or hydrochloric acid, to prepare respectively the lactate or hydrochloride salt of 3,6-dimethyl-5-(4-pyridinyl)-2(1H)-pyridinone in aqueous solution.

D-2. <u>5-(4-Methoxyphenyl)-3,6-dimethyl-2(1H)-pyridinone</u> - A mixture containing 20 g of 3,4-dihydro-5-(4-methoxyphenyl)-3,6-dimethyl-2(1H)-pyridinone, 50 ml of dimethylsulfoxide and 6.1 g of sulfur was heated in an

oil bath at 165-175°C. for 90 minutes. The hot reaction mixture was then poured into 400 ml of ice cold water and the resulting yellow precipitate was collected, washed with water, air-dried, recrystallized from isopropyl alcohol-ether using decolorizing charcoal and dried at 100°C. for two days to yield 8.3 g of 5-(4-methoxyphenyl)-3,6-dimethyl-2(1H)-pyridinone, m.p. 190-192°C.

D-3. <u>5-(4-Hydroxyphenyl)-3,6-dimethyl-2(1H)-pyridinone</u> - A mixture containing 5 g of 5-(4-methoxyphenyl)-3,6-dimethyl-2(1H)-pyridinone and 125 ml of 48% hydrogen bromide was refluxed for five hours and then evaporated to dryness on a rotary evaporator. The residue was treated with aqueous ammonium hydroxide and then reacidified using acetic acid. The yellow solid was collected, washed with water, recrystallized from ethanol and dried at 90°C. to yield 3.4 g of 5-(4-hydroxyphenyl)-3,6-dimethyl-2(1H)-pyridinone, m.p. 276-278°C.

D-4. <u>4,6-Dimethyl-5-(4-pyridinyl)-2(1H)-pyridinone</u>, alternatively named 2,4-dimethyl-[3,4'-bipyridin]-6(1H)-one - A 2 g portion of 1-(4-pyridinyl)-2-propanone was dissolved in 5 ml of methanesulfonic acid, to this solution was added 1 g of acetoacetamide followed by 8 g of polyphosphoric acid. This reaction mixture was then heated at 120-130°C. for 90 minutes and then poured onto ice. The resulting aqueous solution was treated with ammonium hydroxide to a pH of 8. The alkaline mixture was extracted three times with ethyl acetate and the

combined ethyl acetate extracts were concentrated *in vacuo* to dryness. The solid residue was slurried with ether, and, the resulting white solid was collected and washed successively with acetonitrile and ether to yield 650 mg of 4,6-dimethyl-5-(4-pyridinyl)-2(1H)-pyridinone. This reaction was repeated using 16 g of 1-(4-pyridinyl)-2-propanone, 8.1 g of acetoacetamide, 40 ml of methanesulfonic acid and 70 g of polyphosphoric acid. There was thus obtained 9 g of 4,6-dimethyl-5-(4-pyridinyl)-2(1H)-pyridinone which was combined with the above 650 mg and another 300 mg of sample obtained from another run and the combined material was recrystallized from dimethylformamide, washed successively with acetonitrile and ether, and dried in a vacuum oven at 40°C. to produce 8.0 g of 4,6-dimethyl-5-(4-pyridinyl)-2(1H)-pyridinone, m.p. 271-272°C.

Acid-addition salts of 4,6-dimethyl-5-(4-pyridinyl)-2(1H)-pyridinone are conveniently prepared by adding to a mixture of 1 g of 4,6-dimethyl-5-(4-pyridinyl)-2(1H)-pyridinone in about 20 ml of aqueous methanol the appropriate acid, e.g., hydrochloric acid, methanesulfonic acid, concentrated sulfuric acid, concentrated phosphoric acid, to a pH of about 2 to 3, chilling the mixture after partial evaporation and collecting the precipitated salt, e.g., hydrochloride, methanesulfonate, sulfate, phosphate, respectively. Also, the acid-addition salt is conveniently prepared in aqueous solution by adding to water with stirring a molar equivalent quantity each of 4,6-dimethyl-5-(4-pyridinyl)-2(1H)-pyridinone and the appropriate acid, e.g., lactic acid or hydrochloric acid, to prepare respectively the lactate or hydrochloride salt of 4,6-dimethyl-5-(4-pyridinyl)-2(1H)-pyridinone in aqueous solution.

D-5. <u>6-Methyl-5-(4-pyridinyl)-2(1H)-pyridinone</u> - A mixture containing 9.4 g of 3,4-dihydro-6-methyl-5-(4-pyridinyl)-2(1H)-pyridinone, 40 ml of an eutectic mixture of diphenyl and diphenyl ether and 1.6 g of sulfur was heated in an oil bath at 195-200°C. for 4 and 1/2 hours and then allowed to cool to room temperature. The reaction mixture was partitioned between 200 ml of aqueous hydrochloric acid (50 ml of 6N hydrochloric acid and 150 ml of water) and 150 ml of ether. The aqueous layer was separated, treated with decolorizing charcoal and filtered and the filtrate concentrated on a rotary evporator to a volume of about 35 ml. The concentrated solution was neutralized by adding aqueous ammonium hydroxide and then reacidified by adding acetic acid. The crystalline precipitate was collected, washed with water and dried at 85°C. to yield 8.1 g of 6-methyl-5-(4-pyridinyl)-2(1H)-pyridinone, m.p. 285-288°C.

Following the procedure described in Example D-1 using in place of 3,4-dihydro-3,6-dimethyl-5-(4-pyridinyl)-2(1H)-pyridinone a corresponding molar equivalent

quantity of the appropriate 3,4-dihydro-3-$R_1$-4-$R_2$-5-Q-6-R-2(1H)-pyridinone, it is contemplated that the corresponding 3-$R_1$-4-$R_2$-5-Q-6-R-2(1H)-pyridinones of Examples D-6 through D-18 can be obtained.

D-6. 4,6-Dimethyl-5-(4-pyridinyl)-2(1H)-pyridinone, using 3,4-dihydro-4,6-dimethyl-5-(4-pyridinyl)-2(1H)-pyridinone.

D-7. 5-(4-Hydroxyphenyl)-6-methyl-2(1H)-pyridinone, using 3,4-dihydro-5-(4-hydroxyphenyl)-6-methyl-2(1H)-pyridinone.

D-8. 5-(4-Methoxyphenyl)-6-methyl-2(1H)-pyridinone, using 3,4-dihydro-5-(4-methoxyphenyl)-6-methyl-2(1H)-pyridinone.

D-9. 5-(4-Hydroxyphenyl)-3,6-dimethyl-2(1H)-pyridinone, using 3,4-dihydro-5-(4-hydroxyphenyl)-3,6-dimethyl-2(1H)-pyridinone.

D-10. 3,4,6-Trimethyl-5-(4-pyridinyl)-2(1H)-pyridinone, using 3,4-dihydro-3,4,6-trimethyl-5-(4-pyridinyl)-2(1H)-pyridinone.

D-11. 6-Methyl-5-(3-pyridinyl)-2(1H)-pyridinone, using 3,4-dihydro-6-methyl-5-(3-pyridinyl)-2(1H)-pyridinone.

D-12. 6-Ethyl-3-methyl-5-(4-pyridinyl)-2(1H)-pyridinone, using 6-ethyl-3,4-dihydro-5-(4-pyridinyl)-2(1H)-pyridinone.

D-13. 6-Isopropyl-3-methyl-5-(4-pyridinyl)-2(1H)-pyridinone, using 3,4-dihydro-6-isopropyl-3-methyl-5-(4-pyridinyl)-2(1H)-pyridinone.

D-14. 6-n-Butyl-3-methyl-5-(4-pyridinyl)-2(1H)-pyridinone, using 6-n-butyl-3,4-dihydro-3-methyl-5-(4-pyridinyl)-2(1H)-pyridinone.

D-15. 3,4,6-Trimethyl-5-(2-methyl-4-pyridinyl)-2(1H)-pyridinone, using 3,4-dihydro-3,4-6-trimethyl-5-(2-methyl-4-pyridinyl)-2(1H)-pyridinone.

D-16. 6-Ethyl-3-methyl-5-(2,6-dimethyl-4-pyridinyl)-2(1H)-pyridinone, using 6-ethyl-3,4-dihydro-3-methyl-5-(2,6-dimethyl-4-pyridinyl)-2(1H)-pyridinone.

D-17. 6-Ethyl-5-(3-methoxyphenyl)-3-methyl-2(1H)-pyridinone, using 6-ethyl-3,4-dihydro-5-(3-methoxyphenyl)-3-methyl-2(1H)-pyridinone.

D-18. 6-Ethyl-5-(3-hydroxyphenyl)-3-methyl-2(1H)-pyridinone, using 6-ethyl-3,4-dihydro-5-(3-hydroxyphenyl)-3-methyl-2(1H)-pyridinone.

Following the procedure described in Example D-3 but using in place of 5-(4-methoxyphenyl)3,6-dimethyl)-2(1H)-pyridinone a molar equivalent quantity of the appropriate 3-$R_1$-4-$R_2$-5-(methoxyphenyl)-2(1H)-pyridinone, it is contemplated that the corresponding 3-$R_1$-4-$R_2$-5-(hydroxyphenyl)-2(1H)-pyridinones of Examples D-19 and D-22 can be obtained.

D-19. 5-(4-Hydroxyphenyl)-6-methyl-2(1H)-pyridinone, using 5-(4-methoxyphenyl)-6-methyl-2(1H)-pyridinone.

D-20. 6-Ethyl-5-(3-hydroxyphenyl)-3-methyl-2(1H)-pyridinone, using 6-ethyl-5-(3-methoxyphenyl)-3-methyl-2(1H)-pyridinone.

D-21. 6-Ethyl-5-(3-hydroxyphenyl)-4-methyl-2(1H)-pyridinone, using 6-ethyl-5-(3-methoxyphenyl)-4-methyl-2(1H)-pyridinone.

D-22. 5-(4-Hydroxyphenyl)-4,6-dimethyl-2(1H)-pyridinone, using 5-(4-methoxyphenyl)-4,6-dimethyl-2(1H)-pyridinone.

Following the procedure described in Example D-4 but using in place of 1-(4-pyridinyl)-2-propanone a molar equivalent quantity of the corresponding 1-Q-2-alkanone, it is contemplated that the corresponding 4-methyl-5-Q-6-R-2(1H)-pyridinones of Examples D-23 through D-27 can be obtained.

D-23. 4,6-Dimethyl-5-(3-pyridinyl)-2(1H)-pyridinone, using 1-(3-pyridinyl)-2-propanone.

D-24. 6-Ethyl-4-methyl-5-(2-methyl-4-pyridinyl)-2(1H)-pyridinone, using 1-(2-methyl-4-pyridinyl)-2-butanone.

D-25. 4,6-Dimethyl-5-(2,6-dimethyl-4-pyridinyl)-2(1H)-pyridinone, using 1-(2,6-dimethyl-4-pyridinyl)-2-propanone.

D-26. 5-(4-Methoxyphenyl)-4,6-dimethyl-2(1H)-pyridinone, using 1-(4-methoxyphenyl)-2-propanone.

D-27. 6-Ethyl-5-(3-methoxyphenyl)-4-methyl-2(1H)-pyridinone, using 1-(3-methoxyphenyl)-2-butanone.

The utility of the compounds of Formula I where Q is 4(or 3)-hydroxyphenyl, 4(or 3)-pyridinyl or 4(or 3)-pyridinyl having one or two lower-alkyl substituents (or pharmaceutically acceptable acid-addition salts when Q is a pyridinyl substituent) as cardiotonic agents is demonstrated by their effectiveness in standard pharmacological test procedures, for example, in causing a significant increase in contractile force of the isolated cat or guinea pig atria and papillary muscle and/or in causing a significant increase in the cardiac contractile force in the anesthetized dog with low or minimal changes in heart rate and blood pressure. Detailed descriptions of these test procedures appear in U.S. Patent 4,072,746.

When tested by said isolated cat or guinea pig atria and papillary muscle procedure, the compounds of Formula I of the invention or said salts thereof at doses of 1 (3,4-dihydro compounds of Formula I only), 3, 10, 30 and/or 100 µg/ml., were found to cause significant increases, that is, greater than 25% (cat) or 30% (g. pig) in papillary muscle force and significant increases, that is greater than 25% (cat) or 30% (g. pig) in right atrial force, while causing a lower percentage increase (about one-half or less than the percentage increase in right atrial force or papillary muscle force) in right atrial rate. Because of the lower control active tensions of guinea pig tissues, the percent change from control values of both rate and force responses is elevated slightly, i.e., 5%. Thus, whereas cardiotonic activity is ascertained with a papillary muscle force or

right atrial force increase of 26% and greater in the cat test, corresponding activity in the guinea pig test is designated with a papillary muscle force or right atrial force increase of 31% or greater. For example, illustrative cat papillary muscle and right atrial force increases for a 3,4-dihydro compound of the invention are: 175% and 64% at 100 µg/ml, 129% and 53% at 30 µg/ml, and 109% and 51% at 10 µg/ml for the compound of Example C-2. Further, illustrative guinea pig papillary muscle and right atrial force increases for other 3,4-dihydro compounds of the invention are: 100% and 118% at 100 µg/ml, 131% and 98% at 30 µg/ml and 100% and 65% at 10 µg/ml for the compound of Example C-1; 157% and 161% at 10 µg/ml, 127% and 73% at 3 µg/ml and 52% and 39% at 1 µg/ml for the compound of Example C-4; 104% and 87% at 10 µg/ml, and 64% and 72% at 3 µg/ml for the compound of Example C-6; and, 104% and 132% at 10 µg/ml for the compound of Example C-8. Similarly, for example, illustrative guinea pig papillary muscle and right atrial force increases for 3,4-unsaturated compounds of the invention are: 119% and 108% at 30 µg/ml, 155% and 103% at 10 µg/ml and 75% and 55% at 3 µg/ml for the compound of Example D-1; 103% and 263% at 100 µg/ml, 86% and 145% at 30 µg/ml and 73% and 115% at 10 µg/ml for the compound of Example D-4; and, 105% and 70% at 10 µg/ml, and 98% and 82% at 3 µg/ml for the compound of Example D-3.

When tested by said anesthetized dog procedure, the 3,4-dihydro compounds of the invention or said salts thereof at doses of 0.10, 0.30, 1.0 and/or 3.0 mg/kg administered intravenously were found to cause significant increases, that

is, 25% or greater, in cardiac contractile force or cardiac contractility with lower changes in heart rate and blood pressure. For example, the compound of Example C-4 was found to cause respective increases of 51% and 87% in contractile force (cf) at doses of 0.10 and 0.30 mg/kg; and, the compound of Example C-6 was found to cause respective cf increases of 41%, 77% and 107% at doses of 0.3, 1.0 and 3.0 mg/kg.

In clinical practice said compound will normally be administered orally or parenterally in a wide variety of dosage forms. Solid cardiotonic compositions for oral administration include compressed tablets, pills, powders and granules. In such solid compositions, at least one of the active compounds is admixed with at least one inert diluent such as starch, calcium carbonate, sucrose or lactose. These compositions can also contain additional substances other than inert diluents, e.g., lubricating agents, such as magnesium stearate, talc and the like.

Liquid cardiotonic compositions for oral administration include pharmaceutically acceptable emulsions, solutions, suspensions, syrups and elixirs containing inert diluents commonly used in the art, such as water and liquid paraffin. Besides inert diluents such compositions can also contain adjuvants, such as wetting and suspending agents, and sweetening, flavoring, perfuming and preserving agents. According to the invention, the compounds for oral administration also include capsules of absorbable material, such as gelatin, containing said active component with or

without the addition of diluents or excipients.

Preparations comprising compounds of the invention for parenteral administration include sterile aqueous, aqueous-organic and organic solutions, suspensions and emulsions. Examples of organic solvents or suspending media are propylene glycol, polyethylene glycol, vegetable oils such as olive oil and injectable organic esters such as ethyl oleate. These compositions can also contain adjuvants such as stabilising, preserving, wetting, emulsifying and dispersing agents.

They can be sterilized, for example, by filtration through a bacteria-retaining filter, by incorporation of sterilising agents in the compositions, by irradiation or by heating. They can also be manufactured in the form of sterile solid compositions which can be dissolved in sterile water or some other sterile injectable medium immediately before use.

The percentage of active component in the said cardiotonic composition and its concentration when increasing cardiac contractility can be varied so that a suitable dosage is obtained. The dosage administered to a particular patient is variable, depending upon the clinician's judgement using as the criteria: the route of administration, the duration of treatment, the size and condition of the patient, the potency of the active component and the patient's response thereto. An effective dosage amount of active component can thus only be determined by the clinician considering all criteria and utilizing his best judgement on the patient's behalf.

CLAIMS

1. A compound having the Formula (I):

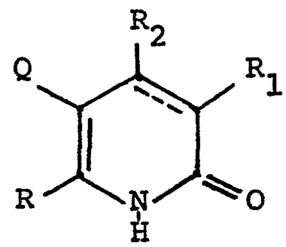

where there is either a single bond between the 3- and 4-positions of the pyridinone ring with hydrogen atoms at said positions (3,4-dihydro) or a double bond between said positions, Q is 4(or 3)-hydroxyphenyl, 4(or 3)-methoxyphenyl, 4(or 3)-pyridinyl or 4(or 3)-pyridinyl having one or two lower-alkyl substituents, $R_1$ and $R_2$ are each hydrogen or methyl but in the event there is a double bond between the 3- and 4-positions of the pyridinone ring, at least one of $R_1$ and $R_2$ is methyl, and R is lower-alkyl, or an acid-addition salt thereof when Q is one of the pyridinyl radicals defined above.

2. A compound according to claim 1, where there is a single bond between the 3- and 4-positions, Q is 4(or 3)-pyridinyl, R is methyl or ethyl and at least one of $R_1$ or $R_2$ is methyl.

3. 3,4-Dihydro-3,6-dimethyl-5-(4-pyridinyl)-2(1H)-pyridinone or acid-addition salt thereof.

4. 3,4-Dihydro-5-(4-hydroxyphenyl)-6-methyl-2(1H)-pyridinone.

5. A compound according to claim 1, where there is a double bond between the 3- and 4-positions, Q is 4(or 3)-pyridinyl and R is methyl or ethyl, $R_1$ is methyl and $R_2$ is hydrogen.

6. 3,6-Dimethyl-5-(4-pyridinyl)-2(1H)-pyridinone or acid-addition salt thereof.

7. 5-(4-Hydroxyphenyl)-3,6-dimethyl-2(1H)-pyridinone.

8. A process for preparing a compound according to claim 1, which comprises:

a) reacting a 2-$R_1$-3-$R_2$-4-Q-4-(RCO)butanenitrile of

the formula $R-\overset{O}{\overset{\|}{C}}-\underset{Q}{CH}-\underset{R_2}{CH}-\underset{R_1}{CH}-CN$ with a strong acid to produce a 3,4-dihydro compound of Formula I,

b) reacting a lower-alkyl 2-$R_1$-3-$R_2$-4-Q-4-(RCO) butanoate having the formula $R-\overset{O}{\overset{\|}{C}}-\underset{Q}{CH}-\underset{R_2}{CH}-\underset{R_1}{CH}-COOR_3$ with ammonia or source thereof where $R_3$ is lower-alkyl to produce a 3,4-dihydro compound of Formula I,

c) reacting an α-$R_1$-β-$R_2$-acrylamide of the formula $R_2-CH=C(R_1)-CONH_2$ in an inert solvent in the presence of a strong base to produce a 3,4-dihydro compound of Formula I or

d) heating a mixture of a 1-Q-2-alkanone of the formula $Q-CH_2-C(=O)R$ and aceto-acetamide with polyphosphoric acid to produce a compound of the Formula I having a double bond between the 3- and 4-positions where $R_1$ is hydrogen and $R_2$ is methyl,

and where R, $R_1$, $R_2$ and Q are as defined in claim 1, except that Q does not include 4(or 3)-hydroxyphenyl and $R_1$ and $R_2$ are limited as recited in process d (above);

and, if desired, heating a 3,4-dihydro compound of Formula I obtained according to any of processes a), b) or c) in a suitable inert solvent in the presence of sulfur to produce a corresponding compound of Formula I having a double bond between the 3- and 4-positions,

and, if desired, converting a compound of Formula I obtained where Q is 4(or 3)-methoxyphenyl to the corresponding compound where Q is 4(or 3)-hydroxyphenyl,

and, if desired, converting a basic compound obtained where Q is one of the above defined pyridinyl radicals to an acid-addition salt thereof.

9. A cardiotonic composition for increasing cardiac contractility, said composition comprising a pharmaceutically acceptable carrier and, as the active component thereof, a cardiotonically effective amount of the compound having the Formula I according to any one of claims 1-7, where Q is

limited to 4(or 3)-hydroxyphenyl, 4(or 3)-pyridinyl or 4(or 3)-pyridinyl having one or two lower alkyl substituents, or a pharmaceutically acceptable acid-addition salt thereof.

10. A process for preparing a 3-$R_1$-4-$R_2$-5-Q-6-R-2(1H)-pyridinone having the formula

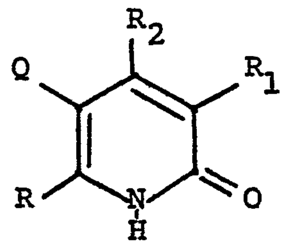

where $R_1$ and $R_2$ are each hydrogen or methyl, R is lower-alkyl, and Q is 4(or 3)-hydroxyphenyl, 4(or 3)-methoxyphenyl, 4(or 3)-pyridinyl or 4(or 3)-pyridinyl having one or two lower-alkyl substituents, which comprises heating a 3,4-dihydro-3-$R_1$-4-$R_2$-5-Q-6-R-2(1H)-pyridinone having the formula

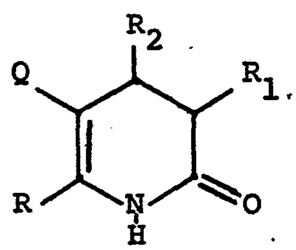

in a suitable inert solvent in the presence of sulfur, wherein $R_1$, $R_2$, R and Q are as defined above except that Q does not include 4(or 3)-hydroxyphenyl, and, if desired, converting said 3-$R_1$-4-$R_2$-5-Q-6-R-2(1H)-pyridinone where Q is 4(or 3)-methoxyphenyl to the corresponding compound where Q is 4(or 3)-hydroxyphenyl.

0109628

European Patent Office

# EUROPEAN SEARCH REPORT

Application number

EP 83 11 1303

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | FR-A-2 470 124 (STERLING DRUG INC.)<br>* Pages 53-55 * | 1,9 | C 07 D 213/64<br>C 07 D 401/04<br>A 61 K 31/44<br>C 07 D 213/57<br>C 07 D 213/55 //<br>(C 07 D 401/04<br>C 07 D 213/00<br>C 07 D 211/00 ) |
| Y,D | US-A-3 718 743 (TSUNG-YING SHEN)<br>* Claim 18; example 10 * | 1 | |
| Y | EP-A-0 060 071 (ELI LILLY)<br>* Page 39; example 34 * | 1 | |
| Y,D<br>P | EP-A-0 074 091 (STERLING DRUGS INC.)<br>* Pages 1,2 * | 1,9 | |
| | | | TECHNICAL FIELDS SEARCHED (Int. Cl. 3) |
| | | | C 07 D 213/00<br>C 07 D 401/00<br>A 61 K 31/00 |

The present search report has been drawn up for all claims

| Place of search | Date of completion of the search | Examiner |
|---|---|---|
| THE HAGUE | 16-02-1984 | VERHULST W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document